# EUROPEAN PATENT SPECIFICATION

(11) **EP 3 559 256 B1**
(45) Date of publication and mention of the grant of the patent: **07.05.2025**
(21) Application number: 17830021.6
(22) Date of filing: 21.12.2017
(51) Int. Cl.: C12Q 1/68, C12Q 1/6883

(54) **METHOD FOR DETERMINING ATTENTION DEFICIT HYPERACTIVITY**
VERFAHREN ZUR BESTIMMUNG VON AUFMERKSAMKEITSDEFIZIT/HYPERAKTIVITÄTSSTÖRUNG
PROCÉDÉ PERMETTANT DE DÉTERMINER UNE HYPERACTIVITÉ AVEC DÉFICIT DE L'ATTENTION

(30) Priority: 22.12.2016 IT 201600129938
(43) Date of publication of application: 30.10.2019
(73) Proprietor: Istituto Superiore di Sanità, 00161 Roma (IT); Universita' Degli Studi di Teramo, 64100 Teramo (TE) (IT); Universita'Degli Studi di Roma "La Sapienza", 00185 Roma (IT)
(72) Inventor: LAVIOLA, Giovanni, 00161 Roma (IT); ADRIANI, Walter, 00161 Roma (IT); PASCALE, Esterina, 00185 Roma (IT); D'ADDARIO, Claudio, 64100 Teramo (IT)
(74) Representative: Delbarba, Andrea
(86) International application number: PCT/IB2017/058254
(87) International publication number: WO 2018/116228

(56) References cited:
- WO-A1-2014/023852
- YI XU ET AL: "Multiple epigenetic factors predict the attention deficit/hyperactivity disorder among the Chinese Han children", JOURNAL OF PSYCHIATRIC RESEARCH, vol. 64, 1 May 2015 (2015-05-01), GB, pages 40 - 50, XP055380671, ISSN: 0022-3956, DOI: 10.1016/j.jpsychires.2015.03.006
- DADDS MARK R ET AL: "Epigenetic regulation of the DRD4 gene and dimensions of attention-deficit/hyperactivity disorder in children", EUROPEAN CHILD AND ADOLESCENT PSYCHIATRY, HOGREFE & HUBER PUBLISHERS, BERN, CH, vol. 25, no. 10, 20 February 2016 (2016-02-20), pages 1081 - 1089, XP036063346, ISSN: 1018-8827, [retrieved on 20160220], DOI: 10.1007/S00787-016-0828-3
- MÜLLER-VAHL KIRSTEN R ET AL: "Gilles de la Tourette syndrome is associated with hypermethylation of the dopamine D2 receptor gene", JOURNAL OF PSYCHIATRIC RESEARCH, vol. 86, 12 November 2016 (2016-11-12), pages 1 - 8, XP029898290, ISSN: 0022-3956, DOI: 10.1016/J.JPSYCHIRES.2016.11.004

## Description

The present invention as defined by the appended claims relates to a method for determining the severity of Attention-Deficit Hyperactivity Disorder (ADHD) or for determining the effectiveness of a therapy against ADHD, said method being based on measuring the methylation levels of CpG dinucleotides and/or CpG islands in a genomic region of the SLC6A3 gene (solute carrier family 6 member 3), preferably in the 5' UTR.

### PRIOR ART

Attention-Deficit Hyperactivity Disorder (ADHD) is a developmental disorder that affects about 4% of children and may persist until adulthood. Although the biological mechanisms underlying ADHD are not fully known, the central role of the neurotransmitter dopamine - and of pharmacological agents active on its reuptake - in promoting psychomotor stimulation point to the importance of brain reward and reinforcement systems in ADHD.

For this reason, with regard to preclinical research on ADHD, considerable interest has been shown in the study of the dopamine transporter (DAT), which is generally abundant in the striatal and prefrontal areas of the brain. Not coincidentally, DAT is also the target of the well-known pharmacological therapies for ADHD that provide for the use of methylphenidate ("Ritalin").

The presently existing approaches for a functional diagnosis of ADHD include concomitant neuroimaging with positron emission tomography (PET) and functional magnetic resonance imaging (fMR).

Clinical diagnosis of ADHD, by contrast, is currently based on structured interviews or questionnaires.

Dadds et al (2016) discloses that 18 epigenetic sites (CpG sites) methylation level in DRD4 are in significantly associated with ADHD and higher methylation level with severe form of ADHD. The document does not mention DAT (SLC6A3).

Therefore, the currently available diagnostic method for diagnosing ADHD is characterised by a strong degree of uncertainty, which leads as a natural consequence to an exponential increase in the prescription of psychostimulant drugs, whose undesirable/side effects are well known, even where would be no need for them.

In the light of the foregoing, there is a strongly felt need to have a method available which would enable ADHD to be determined in a simple manner, but at the same time with a diagnostic accuracy that the present methods do not offer. However, the development of a simple diagnostic test for confirming a diagnosis, or a prognostic one for evaluating the likelihood of an improvement in the ADHD symptoms, is greatly hindered by the elusive nature of the underlying physiopathology of this deficit.

As a solution to the above-mentioned needs, the present invention as defined by the appended claims proposes the method described below, which is essentially based on the analysis, in particular, the measurement, of the methylation levels of specific genomic regions of the SLC6A3 gene (solute carrier family 6 member 3) coding for the dopamine transporter. In particular, the method of the invention enables the presence, the predisposition and/or the prognosis of said disorder to be determined by measuring the methylation levels of specific CpG islands that fall in the 5' UTR of this gene. In fact, the authors have found for the first time that an altered methylation of these CpGs correlates with the presence and/or predisposition and/or prognosis of this disorder.

The method is a simple and useful tool for diagnosis, for prognosis and/or for monitoring the effectiveness of ADHD treatments and enables this to be done with a diagnostic accuracy that the currently available instruments do not allow.

### DESCRIPTION OF THE FIGURES

The present invention as defined by the appended claims is described in detail below and illustrated by way of non-limiting example also with the aid of the following figures.

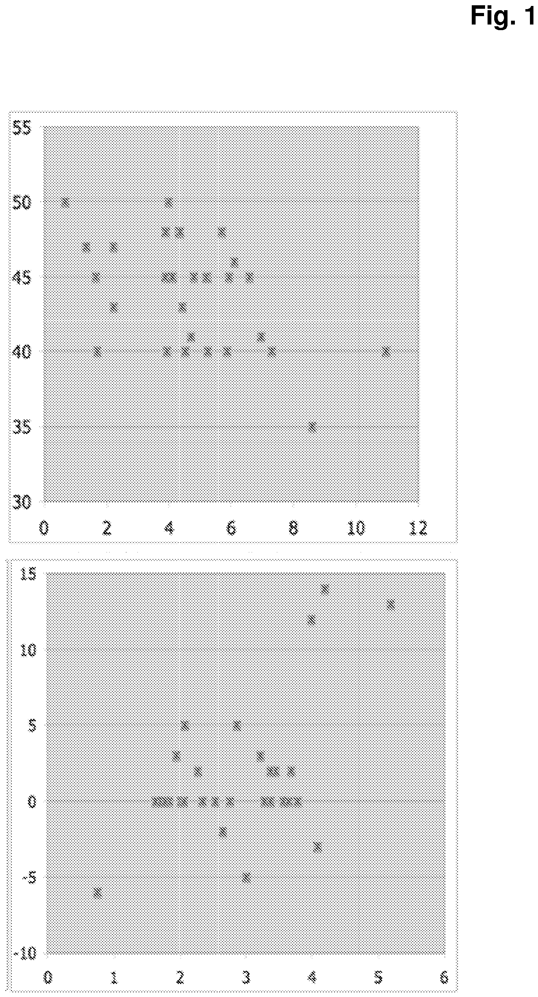

- Figure 1 shows the CGAS score on the Y axis; the methylation of CpG dinucleotide M1 on the X axis (A). Furthermore, (B) shows the delta in the CGAS score (value of the score obtained after 6 weeks of therapeutic treatment minus the value at enrolment) on the Y axis and the methylation of CpG dinucleotide M6.

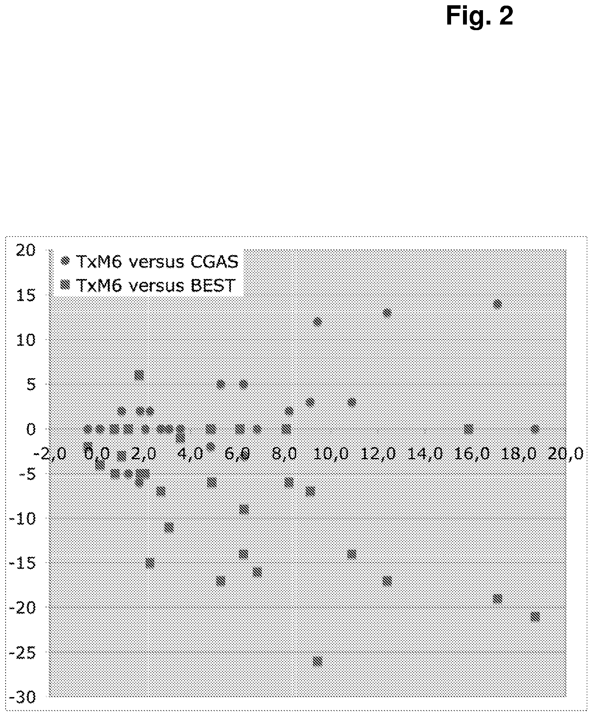

- Figure 2 shows the delta in CGAS scores (value of the score obtained after 6 weeks of therapeutic treatment minus the value at enrolment) on the Y axis and the value TxM6 (the value of the anti-DAT autoantibody titer times the value of the methylation level in position M6) on the X axis.

### DETAILED DESCRIPTION OF THE INVENTION

### DEFINITIONS

In the context of the present method "ADHD", acronym of "attention-deficit hyperactivity disorder", refers to a complex, heterogeneous neurodevelopmental disorder that in 70-80% of the cases coexists with one or more disorders which aggravate the symptoms and make diagnosis and therapy complex. ADHD manifests itself in school age (6-13 years) in a male:female ratio of 4:1, in 4-5% of the population, with symptoms such as hyperactivity, impulsivity and inattention, which are often compounded by oppositionality and thus difficulty in relating with peers and with adults.

In the context of the present method "pyrosequencing" means a rapid, accurate and reproducible DNA sequencing technique. In this context it is applied to the study of DNA methylation. In summary, it is a method of sequencing by synthesis which quantitatively controls the incorporation of nucleotides in real time through enzymatic conversion of the pyrophosphate that is released during nucleotide incorporation as a light signal proportional to the quantity of nucleotides added thanks to the intervention of the enzymes DNA polymerase, ATP sulfurylase, luciferase and apyrase.

The PCR-amplified sequence is incubated as a single strand together with the enzymes and the substrates adenosine phosphosulfate (APS) and luciferin. DNA polymerase catalyzes the addition of a dNTP and, if complementary, inorganic pyrophosphate (PPi) is released. The PPi is transformed into ATP by the action of sulfurylase, using the APS as the substrate. The ATP obtained enables the conversion of luciferin to oxyluciferin by the action of luciferase with the production of a light signal that is detected by a special photosensitive camera. The enzyme apyrase degrades the dNTP that has not been incorporated and the ATP produced by the sulfurylase. Once the degradation is completed, a second dNTP is added to continue the polymerization reaction.

Preferably, in this context the pyrosequencing has been used as an example of a method of quantifying the methylation level following treatment of DNA with bisulfite using the methylation kit of Zymo Research, Orange, CA, USA.

In the context of the present method "DNA methylation" means an epigenetic modification of DNA which comprises the binding of a methyl group (-CH₃) to a cytosine that precedes a guanine in CpG (cytosine-phosphate-guanine) dinucleotides. This modification alters the structure of the DNA in that region, thus modifying accessibility by transcription factors and, consequently, regulating gene expression in this manner. In particular, it is well known that epigenetic modifications of DNA can take place in response to exogenous factors and bring about changes at a phenotypic level. Indeed, at present there is increasingly frequent evidence that the methylation level is associated/correlated with the development of pathologies or predisposition to several pathologies.

In the context of the present method the methylation level is preferably measured by applying the formula Lm=mC/mC+C. Methylation is calculated with this formula for every CpG site, where mC means methylated cytosine and C means unmethylated cytosine. In the present context, when the methylation levels of the specific CpG sites analyzed are lower in a sample than in a reference sample, i.e., a sample isolated from a healthy subject, the sample can be defined as ADHD-positive, i.e., the subject is affected by or has a predisposition to ADHD. Alternatively, the methylation level is calculated following a therapeutic treatment and in this case, therefore, the reference methylation level is the starting one of the same subject with ADHD, or ADHD-positive subject, prior to undertaking the therapy.

Any method useful for measuring the methylation levels of CpG dinucleotides may be used for the purposes of the present method

In the context of the present method "CpG or CpG island" means a cytosine-guanine dinucleotide or a region of the genome that is particularly rich in guanine and cytosine, that is, the G+C content is higher than average. CpG islands are generally characterized by a length of about 1 kb (kilobase) and usually map (i.e., fall) in the promoter region of 60-70% of the genes. Within these dinucleotides or these regions, the majority of CpG pairs are chemically modified by methylation of carbon in position 5 (C5) of the cytosine ring. An abnormal methylation of these regions or of these dinucleotides in proximity to the transcription starting site often leads to an alteration in gene function, an altered expression of the same and a pathology, as has been demonstrated, for example, for cancer.

In the context of the present method the CpG dinucleotides and/or CpG islands analyzed map on human chromosome 5, in the chromosome region comprised between the nucleotides 1,392,790 and 1,445,430 with respect to the human genome 19 (hg19), that is, having these chromosomal coordinates. This chromosome region comprises the chromosome region of the entire SLC6A3 gene. More in particular, the exact positions of these CpGs are shown in Table I.

**Table I**

| | Name | Chromosomal coordinate |
|---|---|---|
| CpG 1 | M1 | 1,444,716 |
| CpG 2 | M2 | 1,444,713 |
| CpG 3 | M3 | 1,444,710 |
| CpG 4 | M5 | 1,444,694 |
| CpG 5 | M6 | 1,444,692 |
| CpG 6 | M7 | 1,444,685 |

The CpG dinucleotides and/or CpG islands analyzed preferably fall in SEQ ID NO: 1, preferably in SEQ ID NO: 2, more preferably in SEQ ID NO: 3. SEQ ID NO: 1-3 are subregions of the chromosome region comprised between the above-mentioned nucleotides 1,392,790 and 1,445,430. In particular, SEQ ID NO: 1 corresponds to the sequence comprised between TSS (transcription starting site) and ATG (translation starting triplet), i.e., the chromosome region (human chromosome 5 - hg19) comprised between chromosomal coordinates 1443195 and 1445430. SEQ ID NO: 2 corresponds to the specific CpG island considered in the example, i.e., the chromosome region (human chromosome 5 - hg19) comprised between chromosomal coordinates 1444634 and 1445075. SEQ ID NO: 3 corresponds to the specific sequence analyzed, i.e., the chromosome region (human chromosome 5 - hg19) comprised between chromosomal coordinates 1444684 and 1444718.

SEQ ID NO: 1-3 are shown in Table II. For the purposes of the present method sequences characterized by an identity comprised between 80-99.9% must be considered as included in the present description.

These sequences can be considered as included within the promoter region, in particular in the 5' UTR of the SLC6A3 gene.

In the context of the present method "promoter" means a DNA sequence containing sequences capable of binding transcription factors and generally positioned upstream of the coding region. In particular, the CpGs that are informative for the purposes of the present method are in a particular region known as 5' UTR, or 5' untranslated region, i.e., in a region that is not canonical for CpGs and their epigenetic modification. It is a portion of DNA downstream (at 3') of the transcription starting site (TSS) but upstream (at 5') of the translation starting site (ATG). Therefore, it is a sequence that is transcribed into mRNA but does not become part of the amino acid sequence of the protein.

In the context of the present method SLC6A3 is an acronym of solute carrier family 6 (neurotransmitter transporter, dopamine), member 3. The human gene codes for the dopamine transporter. Alternative names for this gene are DAT1, PKDYS and DAT. It is located on chromosome 5: 1,392,790-1,445,430 and is made up of 59639 pairs of bases (NCBI Reference Sequence: NG_0158851). The human DAT gene has a polymorphic region in the 3' UTR region of the gene made up of a 40-bp variable number of tandem repeats (VNTR) which generates from 3 to 11 different alleles. The alleles with 9 and 10 repeats are the most frequent in the population. This polymorphism is capable of modulating gene transcription. In particular, the 10-repeat allele is associated with high levels of expression of the DAT gene.

A first aspect of the present invention as defined by the appended claims relates to a method for determining the severity of Attention-Deficit Hyperactivity Disorder (ADHD), or for determining the effectiveness of a therapy against ADHD,

In an embodiment, the methylation level is determined preferably by means of the formula mC/(mC+C), where mC means methylated cytosine and C means unmethylated cytosine.

The ADHD-positive sample is considered severe if the methylation level measured for said CpG having coordinate 1,444,716 (M1) in said sample is higher than the methylation level measured for an ADHD reference sample defined as not severe according to the criteria established by DSM IV-TR (American Psychiatric Association, 2000). In particular, in this context, an ADHD-positive subject is defined as severe when he or she is incapable of functionally and constructively integrating into a number of social contexts (school, family, sports activities) due to the simultaneous presence of more than one symptom selected from the standard ones of hyperactivity, impulsivity and inattention and the comorbid symptoms of oppositionality, aggressiveness and relational disorder. Alternatively, an ADHD-positive subject is defined as severe when it is not possible to train him or her to contain the above-mentioned symptoms using approaches that improve his or her capacity for self-control without the simultaneous need for a pharmacological aid which usually consists of psychostimulating agents, usually methylphenidate or amphetamine derivatives.

The therapy (therapeutic treatment) against ADHD is considered effective if the methylation level measured for said at least one CpG dinucleotide or said at least one CpG island, preferably the CpG dinucleotide having chromosome coordinate 1,444,692 (M6) and/or 1,444,713 (M2), in a sample isolated from an ADHD-positive individual is higher following the therapeutic treatment than the methylation level measured in the same sample before said therapeutic treatment. An effective therapy means one or more approaches that improve his or her capacity for self-control, enabling him or her to integrate more functionally and constructively into a number of social contexts (school, family, sports activities), with or also without a concomitant pharmacological aid.

The method is a method carried out in vitro on a biological sample isolated from a subject suspected of having ADHD. The method thus serves to determine whether said sample is ADHD-positive or not.

The reference sample is the one isolated from a healthy individual, i.e., one who does not have ADHD, in other words ADHD-negative.

A reduction in the methylation levels of specific CpGs in the promoter region of the SLC6A3 gene is associated with the presence of ADHD, i.e., with the severity. preferably of the symptoms, and/or predisposition towards this pathology. Pathology is used in this context also as a synonym of syndrome or deficit. In this context, associated is synonymous with correlated or associable or correlatable.

The method can similarly be used to determine the effectiveness of a therapy for ADHD, i.e., whether the symptoms of an individual with ADHD improve as a result of a pharmaceutical and/or cognitive-behavioural treatment, In this case, the methylation level of at least one CpG or CpG island is measured, as described above, in a sample isolated from an ADHD-positive individual; in the case of the determination of therapeutic effectiveness, the sample is isolated from the ADHD-positive individual before the individual has had the therapeutic treatment. This will be the reference value. The methylation level will subsequently be re-measured in the same individual with the same method as described above following the therapeutic treatment. An increase in the methylation level of at least one CpG or CpG island examined compared to the reference value, i.e., the one measured in the sample isolated from an ADHD-positive individual before that individual has had the therapeutic treatment, is an indicator that the therapy is/was effective, i.e., the individual responds positively to the therapeutic treatment.

The biological sample is isolated from a test subject, i.e., an individual suspected of having or being predisposed to ADHD. Therefore, the method is carried out on a sample isolated from the individual, i.e., it is carried out in vitro, or ex vivo.

Said biological sample can be any biological fluid, preferably selected from: blood, serum, urine, saliva, stools. The sample is preferably taken from the oral cavity; it is preferably buccal. The embodiment that is particularly preferred for the purposes of the present invention envisages that the biological sample is a buccal swab. It is preferably taken from the mouth, preferably inside the cheeks. It preferably comprises exfoliated mucosa cells.

The sample can be rapidly subjected to the nucleic acid extraction step or can be stored, preferably at temperatures enabling the nucleic acids to be preserved.

The step of extracting the nucleic acids from the sample is carried out with the standard techniques available in any laboratory and therefore known to the person skilled in the art. In fact, in the specific context use was made of an extraction kit, namely, the BuccalAmp^{™} DNA Extraction Kit. In this context, extraction is synonymous with isolating or purifying the nucleic acid. Preferably, the nucleic acid is DNA, preferably genomic DNA, more preferably highly purified.

After the step of purifying the nucleic acid, preferably genomic DNA, the latter is subjected to a treatment with bisulfite.

Preferably 100-1000 ng, more preferably about 500 ng of nucleic acid are treated with bisulfite.

Bisulfite converts unmethylated cytosine (C) into uracil (U); as a result, uracil (U) is read as thymine (T) when it is sequenced. Methylated C, by contrast, is not converted and thus there will not be a consequent modification in the sequence. Therefore, any method that enables this objective to be reached can be used as an alternative to the treatment with bisulfite and is therefore included in the present description.

The treatment with bisulfite is carried out with normal laboratory procedures and more preferably, in this context, it is carried out with the EZ DNA Methylation-Gold^{™} Kit, Zymo Research.

The treatment preferably comprises a reaction with bisulfite, preferably sodium bisulfite, at a temperature of about 64 °C, preferably for a time of 2-4 hours. Prior to the reaction, the DNA is preferably denatured with normal procedures providing for a treatment at about 98°C for 10-30 minutes.

At the end of the treatment with bisulfite, the nucleic acid, preferably the genomic DNA, undergoes PCR amplification, preferably by real-time quantitative reverse transcription PCR (RT-qPCR). In this manner, the nucleic acid region of interest is amplified. In this case, use is made of at least one pair of primers designed in such a way as to amplify at least one portion (sequence) of the genomic region comprised between chromosomal coordinates 1,392,790 and 1,445,430 of human chromosome 5, i.e., the genomic region of the entire SLC6A3 gene; the amplified region is preferably selected from SEQ ID NO: 1-3 (see Table II). The region/sequence amplified in this step of the method is called amplicon and said at least one CpG or CpG island falls within this region.

At least one oligonucleotide of the primer pair is distinguished/marked in such a way as to enable monitoring and quantification of the amplified DNA; biotin and/or a fluorophore, for example, may be used as a marker.

The condition of the amplification step provides for the denaturation of the nucleic acid, for example for 10-20, preferably about 15 minutes at a temperature of about 95°C. After the denaturation step, the nucleic acid is subjected to a series of amplification cycles in the presence of the primer pair and polymerase; the number of cycles preferably varies from 40 to 60; it is preferably about 45. Every cycle comprises a denaturation at about 94°C for a few seconds, preferably about 30 sec, a pairing step at a temperature compatible with that of coupling of the selected primers, preferably about 55-58°C, and a lengthening step of a few seconds, preferably about 30 sec at about 72°C. At the end of the cycles the amplified product is preferably subjected to a temperature of about 72°C for 10-20 minutes.

**Table II**

| | |
|---|---|
| SEQ ID NO: 1 | AGCGCTGCGGAGCGGGAGGGAGGCTTCGCGGAACGCTCTCGGCGCCAGG ACTCGCGTGCAAAGCCCAGGCCCGGGCGGCCAGgtgaggccagcgtcgctcgcggcat cggggcgccccgctccttccgcagaccccgaagtggggcgcaggggcgggggccggggaccgggcacagtc tggggtccccgcgtcccgcagaccgcgccgtctccaaagtcgccaacagtcgcgggtgccgagcgccccccga tagcgccacatgggaccctgaggccgtccgaggcgcgaggagggtgcagggctgcccctggccccgctccag gctcagaaccgggtgggcacctggtgcagtcaccggcttaggggacgcgtgggtgtctatggctgtgactcgggg gtcctggtttcttctcgtggaacttaaccctactaggggtgcggcgcatcccagatccgatcggagtgggttttgtaca ccgccgctccatctcgcgggggctttgtctgtgttgggggtggtggcgggcgcgggctgcgcgctggtgctctgggc agggcggggaggccgggcgaggactcgccaggcagcgccgcttcttgttctgggcgcggtgaggaaggacgct ttctaacggccacattttgctgtgtagacccaaaactcgcctctgaggccccgcgttcaggagcggggtcaggtg gccccagggcggcggcgcttgccggagactcgcgagctccgcacccgacgccctctcccaacgcggcctcc tgctcgcgccgcggaacccccttcgtcgggtgttttacccaccggaggggtcgtgccggttgaggttgtcaccgggtg cgtggcatagctcgtgatagctcatgggtgaggttttgtgcaaacttggatgcagggaaagttgcctgttagagcctc cacctgcgacctgcttcagtcgttgtgtgtgtgtgcgcacctgtgtgagtgtgagtgtgtatgtgtgtaagtgtatgtgctc gcctgtgtgtgtgtgagtgtgtatgtgtgtttgtgagtctgtgtgtgtctgtgtgtctgtgtgtgcgtgcgctcgactgaaac acgctgctgctggatccaaatgacagaagtcgccctggctggggcggtgtagacgctcctgctctcctgctcagcgt tgcagggggggtttatgtagccgtttggacaggatttcccgggttaccctgctggcccaggagctagttcccgcgatga agccctgtccatcctccgcccagctctctcacgcggggtggtgccacctgccctaggtggatgtggcttgtacagac actttttgaggaagcagttgtgatggttatgtctaaactctctttacagtggctgattttgcttatataaattttgctctttatta ctgagtataaacaatacaagcccaggcttggtggctcatgcctgtcatctcagcactttgggaggctgaggcagga ggatcgcttgagaccaggagttcaaaaccagccttggcaacaatagtcagaccctgtctctacaaaaaaacaac aacaacaacaaaaaaacacacacaaaaataacttagccggtgctgtggtgcacacctgtagtctcagctgctca ggaggctgaggtgcaaggatcacttgaacctaggaggttgaggcagtgagttgtaatcacaactgtattccatcctg ggtgacagagcgagacctcatgttaaaaaagaaaaaaaaaagaaaaaagaatacagatgaacagtcatgaa gacattattgaatgctcttagaagattgtaaaattgctctctggaagtgtgggggaaggtggaagtgatatccatgca ttgttagtagaaagccacgctagagctcacacagccttgcactttgataggagtggggaggggtgcaggggaag gaggagcaaaccagagtgtctgtcttgaggcctccatgggccagtgccccagccctgtggtgagggctggcactt cccagctcccgtgccccagctgtaccatctcaggcgctgagaacgcacccatcccttcccagaggaatgcccgtg aatgcctcggggctctgccctccgcaccaggtatgtccctagccctggctgctgaattgttgcgttcctgttgtgtgtttat ttttcatattggctgaagACCAAGAGGGAAGAAGCACAGAATTCCTCAACTCCCAGTGT GCCCATG |
| SEQUENCE FROM TSS TO ATG | |
| GENOME LOCATION 5:1443195-1445430 | |
| SEQ ID NO: 2 | accggcttaggggacgcgtgggtgtctatggctgtgactcggggtcctggtttcttctcgtggaacttaaccctact aggggtgcggcgcatcccagatccgatcggagtgggttttgtacaccgccgctccatctcgcggggctttgtct gtgttgggggtggtggcgggcgcgggctgcgcgctggtgctctgggcagggcggggaggccggcgaggac tcgccaggcagcgccgcttcttgttctgggcgcggtgaggaaggacgctttctaacgggccacattttgctgtgta gacccaaaactcgcctctgaggccccgcgttcaggagcggggtcaggtggccccagggcggcggcggcttg ccggagactcgcgagctccgcacccgacgccctctcccaacgcggcctcctgctcgcgccgcggaacccct |
| CpG island EXAMPLE OF GENOME LOCATION 5:1444634-1445075 | |
| SEQ ID NO:3 | |
| MINIMUM SEQUENCE COMPRISING THE CpGs | *g**cg**g**cg**g**cg**gcttgccggagact**cgcg**agctc**cg**c* |
| EXEMPLIFIED IN THE GENOME LOCATION STUDY 5:1444684-1444718 | |

Said at least one CpG or CpG island whose methylation level is analyzed with the method of the invention falls in this region.

Said at least one CpG or CpG island is preferably selected from at least one CpG having a chromosome coordinate (human chromosome 5) selected from: 1,444,716; 1,444,713; 1,444,710; 1,444,694; 1,444,692; 1,444,685 and any combination thereof (see Table I). As already mentioned, the chromosomal coordinates given here make reference to human genome-19 (hg19).

In a particularly preferred embodiment the method provides for the analysis of the methylation levels of all 6 CpGs specified above. In other embodiments, the methylation levels of M1 and/or M2 and/or M6 corresponding, respectively, to the following chromosomal coordinates 1,444,716; 1,444,713 and 1,444,692are analyzed. In other words M1, M2 and M6 are the most informative CpGs.

In particular, a higher methylation level of CpG M1 (chromosome coordinate 1,444,716) in an ADHD-positive sample compared to the methylation level of the same site in an ADHD-positive sample defined as not severe is correlated (associated) with, or is a biomarker of, the clinical severity of ADHD, i.e., severe ADHD symptoms.

In fact, in the examples it has been demonstrated that a high level of methylation at this site measured in subjects with ADHD at the time of enrolment in the study are associated or correlate with a lower CGAS score (R=-0.487). CGAS is a type of clinical questionnaire as noted earlier; in particular, the lower the CGAS score, the more severe the ADHD symptoms are.

Furthermore, the methylation level of CpG M6 (chromosome coordinate 1,444,692) and/or M2 (chromosome coordinate 1,444,713) is correlated (associated) with, or is a biomarker of, the response to the therapy. In particular, high methylation levels of at least one and/or both of these CpGs are a biomarker of a good response to the therapy against ADHD. In other words, if the methylation level measured for at least one and/or both of these CpGs (M6 and/or M2) increases as a result of the therapeutic treatment undertaken by the individual with ADHD, it means that the subject is responding positively to the therapy, i.e., the therapy is effective. In this context, therapy against ADHD means a cognitive-behavioural therapy and/or a pharmacological therapy, for example one based on the administration of psychostimulant drugs, for example like methylphenidate. What is meant in particular is one or more approaches that improve his or her capacity for self-control, enabling him or her to integrate more functionally and constructively into a number of social contexts (school, family, sports activities), with or also without a concomitant pharmacological aid.

In fact, in the example it has been demonstrated that after the therapeutic treatment, the subjects that had a higher methylation level (i.e., lower than that of the control (healthy) subjects, but not as low as that of ADHD-positive subjects defined as severe), of CpG M6 had responded better to the therapy, as demonstrated by the scores obtained with the CGAS questionnaires (R=+0.434). Furthermore, when the delta or difference in the scores of the CGAS and Conners questionnaires before and after the treatment was evaluated, it was demonstrated that the level of M2 is significant in the same manner, i.e., an increase in the methylation level in this CpG (M2) is associated/correlated with a better therapeutic response of the ADHD subject (in the case of the delta - difference between the levels measured before and after the therapeutic treatment - the significant values were R=0.464 for M2 and R=0.540 for M6). Therefore the methylation level of CpG M2 and/or M6 is a biomarker of therapeutic response, i.e., an increase is correlated/associated (correlatable/associable) with a positive therapeutic response of the subject.

A further aspect of the present method relates to the combination of the method with the levels of the anti-DAT autoantibodies, that is, with the titer, preferably measured in the serum, of human anti-DAT (dopamine transporter) autoantibodies, hDAT-AABs (human DAT autoantibodies), i.e., the antibodies produced by the immune system of a subject against his or her own dopamine transporter, preferably as a result of an autoimmune reaction. The hDAT-AAB levels can also be non-pathological (within the norm).

Therefore, according to a preferred embodiment the method comprises a further step of measuring the hDAT autoantibody level/titer, preferably in human serum. The method used for this purpose is preferably the one disclosed in patent application WO/2014/023852 of the authors of the present study.

In any case every alternative method useful for this purpose is to be considered part of the present description.

Alternatively, the antibody level/titer may be predetermined, i.e., it was determined/measured prior to the implementation of the method of the invention.

In particular, the value of the hDAT autoantibody titer/level is multiplied by the methylation level measured with the method of the invention, preferably the value of the hDAT autoantibody titer/level is multiplied by the methylation level measured for said at least one CpG/CpG island present in the amplified region of interest. The CpG is preferably at least one of the six CpGs in Table I, preferably all of the CpGs. For the purposes of the present invention, it is particularly useful to evaluate the index obtained by multiplying the hDAT autoantibody titer/level by the methylation level measured with the method of the invention for CpG M1 (chromosome coordinate 1,444,716 - T (titer) x M1 (methylation at CpG site 1,444,716), and/or M6 (chromosome coordinate 1,444,692- TxM6) and/or M2 (chromosome coordinate 1,444,713 - TxM2).

T (titer) x M1 (methylation at CpG site 1,444,716) is associated/correlated with, or is a biomarker of, the clinical severity of ADHD, preferably in individuals having the DAT 10/10 genotype, i.e., homozygous for the 10-repeat allele of the VNTR. In particular, high methylation levels in this CpG together with high hDAT-AAB titer levels are a biomarker of the clinical severity of ADHD, i.e., of severe ADHD symptoms. In other words, the index obtained from T (titer) x M1 (methylation at CpG site 1,444,716) has a diagnostic value, preferably in individuals having the DAT 10/10 genotype.

Therefore, an increase in the methylation at CpG site M1 and/or the value of TxM1 (hDAT autoantibody titer for the methylation level of the CpG at chromosome (chromosome 5) coordinate 1,444,716) is/are a biomarker for the clinical severity of ADHD, preferably in individuals having the DAT 10/10 genotype.

In fact, the experimental data reported in the example demonstrated that the value of the index TxM1 is negatively correlated with the score obtained with the questionnaire CGAS (R = -0,482).

In a further preferred embodiment the values TxM6 (hDAT autoantibody titer for the methylation level of CpG at chromosome (chromosome 5) coordinate 1,444,692) and/or TxM2 (hDAT autoantibody titer for the methylation level of the CpG at chromosome (chromosome 5) coordinate 1,444,713) are correlated/associated with a better therapeutic response of the ADHD subjects, preferably in subjects with the DAT 9-repeat allele, preferably subjects having the DAT 9/9 or DAT 9/10 genotype (TxM6 is positively correlated with the score obtained with the CGAS questionnaire (R = 0,515). TxM2 is positively correlated with the score obtained with the CGAS questionnaire (R = 0.504); therefore, the indexes TxM6 and/or TxM2 are/is a biomarker of therapeutic response, that is, an increase in the value of at least one of these indexes is correlated/associated (correlatable/associable) with a positive therapeutic response of the subject. A value of TxM6 greater than or equal to 4 is particularly preferred. In fact, in the examples it was demonstrated that subjects who, following therapy, show a value of this index that is greater than or equal to 4 have a good recovery, whereas for values below 4 a recovery is often not observed.

In a further preferred embodiment for the purposes of evaluating the outcome in terms of the therapeutic response of a subject with ADHD, the value of the index Tx(M6+M2) (hDAT autoantibody titer multiplied by the average value of the methylation level of CpGs M6 and M2 having chromosomal (human chromosome 5) coordinates 1,444,692and 1,444,713, respectively). In particular, an increase in the value of this index after a therapeutic program followed by an individual with ADHD, preferably in subjects with the DAT 9-repeat allele, preferably subjects having the genotype DAT 9/9 or DAT 9/10, is a biomarker of therapeutic response, i.e., an increase in the value of this index is correlated/associated (correlatable/associable) with a positive therapeutic response of the subject. In fact, in the example it was demonstrated that the value of this index increases further (and is therefore even more significant) than the single products of TxM2 or TxM6. In the example,

Tx(M6+M2) is positively correlated with the CGAS score (R= 0.523) to a more significant degree than TxM6 (R = 0.515) or TxM2 (R = 0.504).

A further aspect relates to the use of CpG dinucleotides having chromosome coordinate 1,444,716 or the index obtained by multiplying the value of the methylation level of said CpG dinucleotides having chromosome coordinate 1,444,716 by the value of the anti-DAT autoantibody titer as a biomarker of the clinical severity of ADHD, preferably in an individual having a DAT homozygous 10/10 genotype, where said methylation level is preferably measured as previously described.

A further aspect relates to the use of the CpG dinucleotides having a chromosome coordinate selected from 1,444,713, and/or 1,444,692or of the index obtained by multiplying the value of the methylation level of said CpG dinucleotides having a chromosome coordinate selected from 1,444,713, and/or 1,444,692by the value of the anti-DAT autoantibody titer as a biomarker of the effectiveness of the therapy against ADHD, preferably in an individual having a DAT 9-repeat allele, preferably the genotype DAT 9/9 or DAT 9/10, where said methylation level is measured as previously described.

### EXAMPLE

### Samples

Samples were taken both from healthy subjects (n=8) and subjects with ADHD (n=30), aged between 6 and 14 years.

Buccal swabs were used to isolate the DNA. The white terminal part of the sterile, single-use buccal swab was rubbed on the inside of the cheeks for about 60 seconds. The swab was placed back in its cylindrical plastic container, labeled and sent to the laboratory.

### DNA extraction

The BuccalAmp^{™} DNA Extraction Kit (Epicentre) was used to extract DNA, following the instructions provided by the manufacturer. Any other source of DNA can be used.

### Completion of Questionnaires and Measurement of anti-hDAT autoantibodies

The method regarding enrolment in the clinical study, completion of the ADHD scores by means of the relevant questionnaires, and measurement of hDAT-AAB serum levels described in the same were carried out as described in patent application WO/2014/023852.

The questionnaires used are broadly utilized in this technical field and well known. In particular, in this example the following were used:
1) Children's Global Assessment Scale (CGAS), i.e., a numerical scale used by clinicians to compare the general functioning of the under-18 population. The scale goes from 1 to 90 or 1 to 100 and a high score indicates better functioning; and
2) Conners Comprehensive Behavior Rating Scales (CBRS - Conners); this is an instrument used to obtain a broad-spectrum assessment of the behaviour, emotions and social and academic profiles of the young population, aged between 6 and 18 or 8 and 18 years.

The procedures described in patent application WO2014023852 were used to measure the serum levels of hDAT-AABs.

### Treatment with Bisulfite

500 ng of DNA were treated with bisulfite (EZ DNA Methylation-Gold^{™} Kit, Zymo Research) following the guidelines suggested by the manufacturer. 500 ng of DNA were treated with sodium bisulfite (EZ DNA Methylation-Gold^{™} Kit, Zymo Research) following the guidelines suggested by the manufacturer. The DNA, resuspended in 20 µL of nuclease-free H2O, had 130 µL of CT Conversion Reagent added to it and was exposed to the following temperatures:
98° C for 10 minutes
64° C for 2 hours and 30 minutes
4° C

The sample was then loaded in a column (Zymo-Spin^{™} IC) previously balanced with 600 µL of M-Binding Buffer and subjected to centrifugation for 1 min at 10,000 x g. Once the eluate had been removed, 100 µL of M-Wash Buffer were added to the column and it was again centrifuged for 1 min at 10,000 x g. After the addition of 200 µL M-Desulphonation Buffer, the column was incubated for 17 minutes at room temperature. The incubation was followed by a step in the centrifuge (10,000 x g for 1 min). The column was washed twice through the addition of 200 µL of M-Wash Buffer followed by centrifugation (10,000 x g for 1 min). The converted DNA was eluted in 20 µL of nuclease-free water.

### Amplification of the sequence of interest

The converted DNA was amplified using the PyroMark PCR Kit (Qiagen), based on the manufacturer's protocol.

Briefly, 2.5 µL of converted DNA was added to the following reaction mixture:
- 12.5 µL of PCR Master Mix
- 2.5 µL Fw Primer + Rv Primer (a biotin is bound to the 3' end of the fw primer).
- 2.5 µL of CoralLoad Concentrate

The volume of 25 µL was reached by adding nuclease-free H₂O.

The primer pair used for amplification and the sequencing primer were produced by Qiagen (hsI_SLC6A3_01PM Pyromark CpG (PM00022064) assay).

The PCR conditions were the following: 95°C for 15 minutes, followed by 45 cycles of 94°C for 30s, 56°C for 30s, 72°C for 30s, and, finally, 72°C for 10 minutes. The PCR products (234 bp) were analyzed by agarose gel electrophoresis.

Pyrosequencing was used as the standard technique for quantifying methylation after treatment of the DNA with bisulfite, using the methylation kit of Zymo Research, Orange, CA, USA.

The amount of DNA treated with bisulfite and subjected to amplification was 2.5 µl of bisulfite-treated DNA, i.e., 25 ng/ul of DNA.

The amplicon length was 234 bp.

The analysis of the results was conducted using the PyroMark Q24 (Qiagen); the methylation level was analyzed using PyroMark Q24 Software (Qiagen), which calculates the percentage of methylation (mC/(mC + C)) for each CpG site, thus enabling quantitative comparisons (where mC means methylated cytosine and C means unmethylated cytosine)

The potential correlation between the DNA methylation level of 6 specific CpGs and the scores obtained in the clinical questionnaires was subsequently studied.

The clinical questionnaires used in this case were:
1) Children's Global Assessment Scale (CGAS); and
2) Conners Comprehensive Behavior Rating Scales (CBRS - Conners). Table I below shows the 6 CpGs considered.

**Table I**

| | | |
|---|---|---|
| CpG 1 | M1 | 1,444,716 |
| CpG 2 | M2 | 1,444,713 |
| CpG 3 | M3 | 1,444,710 |
| CpG 4 | M5 | 1,444,694 |
| CpG 5 | M6 | 1,444,692 |
| CpG 6 | M7 | 1,444,685 |

In particular, the correlations between the methylation levels of these 6 CpGs and the scores of the Conners and CGAS questionnaires were assessed using 3 methods:
1) by determining the score at the time of enrolment (Table III);
2) by determining the score after 6 weeks of therapeutic treatment, both of a cognitive-behavioural and pharmacological type, for example with psychostimulant drugs such as methylphenidate (Ritalin) (Table IV); and
3) by determining the difference between (delta) the values according to points 1 and 2 in order to provide an index of symptom improvement (Table V).

Table III below shows the correlations between the methylation levels of the 6 CpGs analyzed and the Conners and CGAS scores at the time of enrolment (point 1).

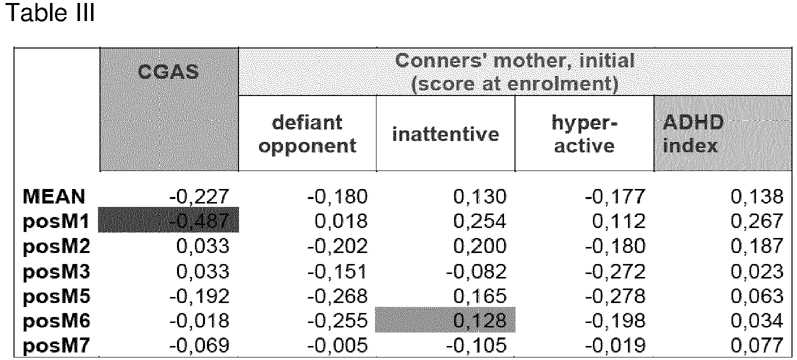

A particularly interesting result emerging from these results is that the methylation level of CpG M1 (5: 1,444,716) is negatively correlated with CGAS (R = -0.487).

In other words, a higher methylation level of CpG M1 is an indicator of a low CGAS score.

It is well known that the worse the symptoms of ADHD are, the lower the CGAS score will be.

Therefore, it is evident from these data that high CpG M1 methylation values are associated with a clinically severe form of ADHD. That is, CpG M1 is a biomarker of the clinical severity of ADHD.

Table III, on the other hand, lists the correlations between the methylation levels of the six CpG sites analyzed and the Conners and CGAS scores after six weeks of the therapeutic treatment.

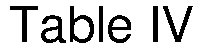

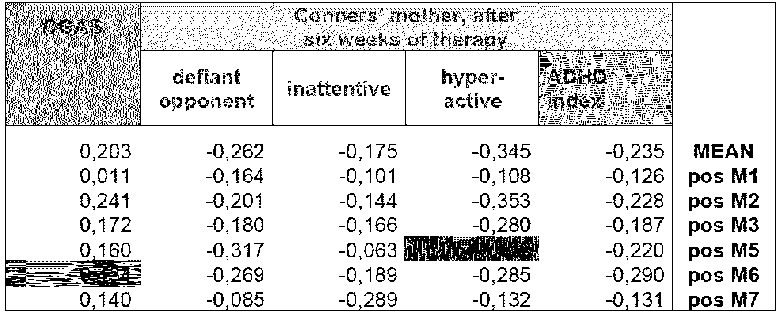

The data shown in the table demonstrate that the methylation level of the M6 site positively correlates with CGAS (R = + 0.434).

In other words, a higher methylation level of CpG M6 correlates with a high CGAS post-therapy score.

It is well known that less severe symptoms of ADHD are associated with a higher CGAS score and given that we are seeing six weeks of therapeutic effectiveness, an increase in the methylation level at the CpG site M6 is associated with, or is a biomarker of, a significantly positive response to the therapy for the treatment of ADHD.

Table V shows the correlations between the methylation levels of the six CpG sites considered and the "delta" in the Conners and CGAS scores, i.e., the difference between the value at six weeks and the one measured at the time of enrolment. The "delta" provides an indicator of the improvement of symptoms observed during the course of the therapy.

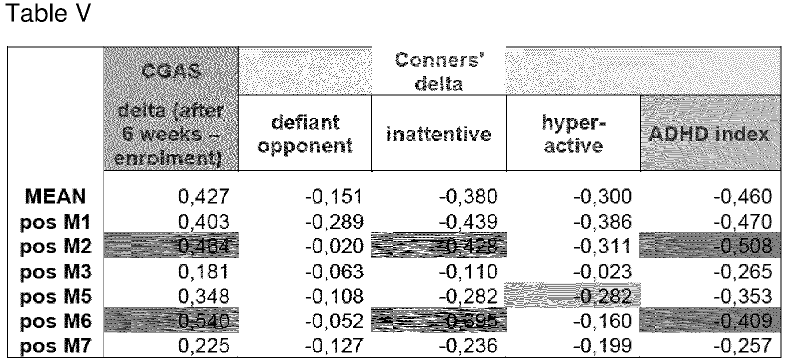

In particular, the results shown in the table demonstrate that the CGAS delta positively correlates with the methylation level of the CpG sites M6 and M2 (R = 0.540; R = 0.464, respectively). Therefore, a better profile, i.e., a reduced severity of ADHD symptoms is associated/associable with a higher CGAS delta, which is indicated by high scores of the methylation levels of the CpG sites M6 and M2 (Fig.1).

This is supported by the 'Conners' delta. In fact, when the child's mother is able to report an improvement, i.e., there is a decrease in the inattention score and an improvement, i.e., a decrease in the ADHD index, the extensions of such improvements are respectively correlated with the methylation levels of the CpG sites M2 (R = - 0.428; R = -0.508) and M6 (R = -0.395; R = -0.409). Therefore, together with high methylation levels at the CpG site M6, high values of the methylation level at the CpG site M2 are also associated with, or are a biomarker of, a significantly positive response to the therapeutic treatment for ADHD.

The correlation between the methylation level of the CpGs considered and the hDAT-AABS (serum autoantibodies of the human dopamine transporter) titer, i.e. a further innovative diagnostic marker for ADHD, which is a measurement of the serum levels of autoantibodies targeting the human dopamine transporter, was also verified.

In this case the data demonstrate that the methylation level of the CpG sites M1 and M6 correlate with the autoantibody titer. In particular, the correlation between the autoantibody titer and methylation level of the CpG site M1 is associated/associable with the clinical severity of ADHD, whereas the correlation between the autoantibody titer and the methylation level of the CpG site M6 is associated/associable with the response to the therapy for the treatment of ADHD.

For this reason, a new index was constructed, consisting in the multiplication of the methylation level of CpG sites M1 and M6 by the autoantibody titer, where said indexes are defined TxM1, TxM6, respectively.

In order to have a more complete characterization, these indexes were assessed for all CpG sites considered, i.e., TxM1, TxM2, TxM3, TxM5, TxM6 and TxM7.

In particular, a calculation was made of the correlation:
a) between these TxM indexes and the CGAS score at enrolment (Table V); and
b) between these TxM indexes and the Conners and CGAS score "delta" (difference between the initial value and the value after six weeks).

In the first analysis, as shown in Table VI, the TxM1 index negatively correlates with CGAS (R = -0.482), i.e., both the high values of the DAT-aABS autoantibody titer and the methylation level of the CpG site M1 are associated/associable with low CGAS scores, or severe symptoms of ADHD.

In conclusion, the TxM1 index is associated/associable with, or is a biomarker of, the clinical severity of ADHD.

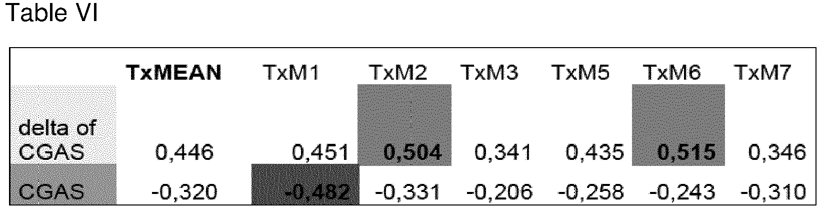

The other analysis performed relates to the TxM indexes and the "delta". The CGAS delta is associated/associable to a significant degree not only with TxM6 but also with TxM2 (R = 0.515; R = 0.504, respectively; see Fig. 2).

Furthermore, the TxM2+6 index, obtained by multiplying the average of the methylation levels of the CpG sites M6 and M2 by the autoantibody titer, correlates or is associated/associable with the CGAS delta to an even more significant degree (R = 0.523; Table VII).

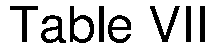

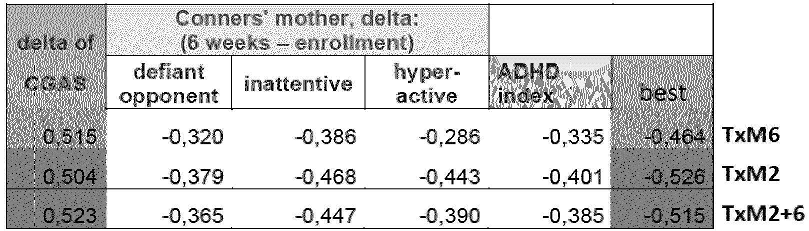

It is interesting to note that, when Fig.1 and Fig. 2 are compared, panel B, it is evident that the individuals who show a good recovery, thus respond to the therapy (CGAS delta > 2), also have a value of TxM6 > 4.

Only four individuals with a value of the TxM6 index > 4 do not show recovery, whereas the remaining individuals with TxM6 < 4 show no recovery or even a worsening of the pathology, with a CGAS delta < 2 and even negative.

Therefore, a value of TxM6 > 4 could be used as a recovery forecast index.

In fact, when mothers were able to report an improvement (for example a decrease) in the inattention score (and this time the correlations are weak as far as the ADHD index is concerned), the entity of such improvements was correlated strongly with TxM2 (R = -0.468; also known as R = -0.443 in hyperactivity) and rather weakly with TxM6 (R = -0.386), but became stronger with TxM2+6 (R = -0.477).

Given that the mothers' assessments in classifying a perceived change as belonging to an 'opponent' vs. 'inattention' vs. 'hyperactive' phenotype cannot be so precise, a further approach was sought, where the most evident change (that is, the greatest negative delta between the three subscales mentioned, for each patient) was used in order to be correlated with TxM2 and TxM6.

The resulting correlations were R = -0.526 and R = -0.464, respectively. Figure 2 (squares) shows a clear idea of this correlation, so that twelve patients with TxM6 > 4 show a decrease (negative change = improvement in symptoms) of more than 5 points in at least one of the Conners' subscales as assessed by mothers.

Therefore, the methylation level of CpG M6, i.e., the CpG having chromosome coordinate 1,444,692, is more in accordance with the opinion of clinical personnel, whereas the methylation level of M2, i.e., the CpG having chromosome coordinate 1,444,713, is more in accordance with the opinion of mothers.

In fact, the CGAS delta is closely correlated with M6 (R = 0.540) and with TxM6 (R = 0.515), while the correlations with regard to "delta " - on the Conners' scale, with regard to data collected from mothers - are significant in the case of the ADHD index (for M2 : R = -0.508) and the best variation (i.e. greatest "delta") in the three Conners' subscales (TxM2 : R = -0.526).

After six weeks of therapy, a better profile (i.e., a reduced severity) of ADHD symptoms (suggested by a higher CGAS delta), together with a specific improvement with respect to inattention (or at least one of the three main symptoms), as observed by mothers, is apparently indexed through the joint use of biomarkers as higher scores of the anti-DAT autoantibody (hDAT - AAB) titer and methylation levels, which can be represented by the M6 site alone or by the M6 and M2 sites together.

## Claims

1. Method for determining the severity of Attention-Deficit Hyperactivity Disorder (ADHD), or for determining the effectiveness of a therapy against ADHD, said method comprising the steps of:
(i) purifying the genomic DNA from an isolated sample that has been obtained from an ADHD-positive individual or from an individual suspected of being ADHD-positive;
(ii) reacting said genomic DNA with bisulfite;
(iii) amplifying, preferably by PCR, more preferably by RT-qPCR, at least one portion of the genomic region comprised between chromosomal coordinates 1,392,790 and 1,445,430 of human chromosome 5 comprising at least one CpG dinucleotide or a CpG island, said coordinates being calculated with respect to human genome 19;
(iv) measuring the methylation level of at least one CpG dinucleotide or at least one CpG island comprised in the amplified region,
wherein said ADHD-positive sample is considered severe if the methylation level measured for said CpG having coordinate 1,444,716 of human chromosome 5 in said sample is higher than the methylation level measured for an ADHD reference sample defined as not severe;
wherein said therapy is considered effective if the methylation level measured, in a sample isolated from an ADHD-positive individual following the therapeutic treatment, for said at least one CpG dinucleotide or said at least one CpG island is higher than the methylation level measured in the same sample before said treatment, wherein said CpG or CpG island is selected from at least one CpG having a chromosome coordinate selected from: 1,444,713 and 1,444,692 of human chromosome 5, wherein said therapy against ADHD comprises a cognitive-behavioral therapy and a pharmacological therapy based on the administration of psychostimulant drugs selected from methylphenidate and amphetamine derivatives.

2. Method according to claim 1, wherein the measurement of the methylation level according to step (iv) is determined by means of the formula mC/(mC+C), wherein mC means methylated cytosine and C means unmethylated cytosine.

3. Method according to claim 1 or 2, wherein said biological sample is a biological fluid selected from the group consisting of: blood, serum, urine, saliva and stools.

4. Method according to claim 3, wherein said biological sample is a buccal swab.

5. Method according to any one of claims 1-4, wherein the amount of genomic DNA ranges between 100 and 1000 nanograms; more preferably it is about 500 nanograms.

6. Method according to any one of claims 1-5, wherein the amplified region is selected from the group consisting of SEQ ID NO: 1-3.

7. Method according to any one of claims 1-6, in combination with the scores of at least one clinical questionnaire and/or the anti-DAT autoantibody titer.

8. Method according to claim 7, wherein the at least one clinical questionnaire is CGAS and/or Conners.

9. Method according to claim 7 or 8, wherein the anti-DAT autoantibody titer is the serum titer.

10. Use of The CpG dinucleotide with chromosomal coordinate 1,444,716 of the human chromosome 5 as a biomarker of the ADHD clinical severity.

11. Use of The CpG dinucleotide according to claim 10, in an individual with a DAT 10/10 homozygous genotype

12. Use of The CpG dinucleotide having a chromosomal coordinate selected from 1,444,713 and 1,444,692; of human chromosome 5 as a biomarker of the effectiveness of the therapy against ADHD, wherein said therapy against ADHD comprises a cognitive-behavioral therapy and a pharmacological therapy based on the administration of psychostimulant drugs selected from methylphenidate and amphetamine derivatives.

13. use of The CpG dinucleotide according to claim 12, in a subject with a DAT 9-repeat allele.

14. Use of The CpG dinucleotide according to claim 12 or 13, wherein the subject has a DAT 9/9 or DAT 9/10 genotype.

## Patentansprüche

1. Verfahren zur Bestimmung des Schweregrads der Aufmerksamkeitsdefizit-/Hyperaktivitätsstörung (ADHS) oder zur Bestimmung der Wirksamkeit einer Therapie gegen ADHS, wobei das Verfahren die folgenden Schritte umfasst:
(i) Reinigen der genomischen DNA aus einer isolierten Probe, die von einem ADHS-positiven Individuum oder von einem Individuum, der im Verdacht steht, ADHS-positiv zu sein, erhalten wurde;
(ii) Umsetzen der genomischen DNA mit Bisulfit;
(iii) Amplifizieren, vorzugsweise durch PCR, bevorzugter durch RT-qPCR, mindestens eines Abschnitts der genomischen Region zwischen den Chromosomenkoordinaten 1.392.790 und 1.445.430 des menschlichen Chromosoms 5, umfassend mindestens ein CpG-Dinukleotid oder eine CpG-Insel, wobei die Koordinaten in Bezug auf das menschliche Genom 19 berechnet werden;
(iv) Messen des Methylierungsgrads mindestens eines CpG-Dinukleotids oder mindestens einer CpG-Insel, die in der amplifizierten Region enthalten ist,
wobei die ADHS-positive Probe als schwerwiegend gilt, wenn der Methylierungsgrad, der für das CpG mit der Koordinate 1.444.716 des menschlichen Chromosoms 5 in der Probe gemessen wurde, höher ist als der Methylierungsgrad, der für eine ADHS-Referenzprobe gemessen wurde, die als nicht schwerwiegend definiert ist;
wobei die Therapie als wirksam gilt, wenn der Methylierungsgrad, der in einer Probe, die von einem ADHS-positiven Individuum nach der therapeutischen Behandlung isoliert wurde, für das mindestens eine CpG-Dinukleotid oder die mindestens eine CpG-Insel gemessen wurde, höher ist als der in derselben Probe vor der Behandlung gemessene Methylierungsgrad, wobei das CpG oder die CpG-Insel aus mindestens einem CpG ausgewählt ist, aufweisend eine Chromosomenkoordinate ausgewählt aus: 1.444.713 und 1.444.692 des menschlichen Chromosoms 5, wobei die Therapie gegen ADHS eine kognitive Verhaltenstherapie und eine pharmakologische Therapie umfasst, die auf der Verabreichung von Psychostimulanzien basiert, die aus Methylphenidat und Amphetaminderivaten ausgewählt sind.

2. Verfahren nach Anspruch 1, wobei die Messung des Methylierungsgrads nach Schritt (iv) mittels der Formel mC/(mC+C) bestimmt wird, wobei mC methyliertes Cytosin bedeutet und C unmethyliertes Cytosin bedeutet.

3. Verfahren nach Anspruch 1 oder 2, wobei die biologische Probe eine biologische Flüssigkeit ist, die aus der Gruppe ausgewählt ist, die aus Blut, Serum, Urin, Speichel und Stuhl besteht.

4. Verfahren nach Anspruch 3, wobei die biologische Probe ein Wangenabstrich ist.

5. Verfahren nach einem der Ansprüche 1-4, wobei die Menge an genomischer DNA im Bereich zwischen 100 und 1000 Nanogramm liegt; bevorzugter beträgt sie etwa 500 Nanogramm.

6. Verfahren nach einem der Ansprüche 1-5, wobei die amplifizierte Region ausgewählt ist aus der Gruppe bestehend aus SEO ID NO: 1-3.

7. Verfahren nach einem der Ansprüche 1-6 in Kombination mit den Werten mindestens eines klinischen Fragebogens und/oder des Anti-DAT-Autoantikörpertiters.

8. Verfahren nach Anspruch 7, wobei der mindestens eine klinische Fragebogen CGAS und/oder Conners ist.

9. Verfahren nach Anspruch 7 oder 8, wobei der Anti-DAT-Autoantikörpertiter der Serumtiter ist.

10. Verwendung des CpG-Dinukleotids mit chromosomaler Koordinate 1.444.716 des humanen Chromosoms 5 als Biomarker des klinischen Schweregrads von ADHS.

11. Verwendung des CpG-Dinukleotids nach Anspruch 10 bei einem Individuum mit einem homozygoten Genotyp DAT 10/10.

12. Verwendung des CpG-Dinukleotids mit einer chromosomalen Koordinate, ausgewählt aus 1.444.713 und 1.444.692 des menschlichen Chromosoms 5 als Biomarker für die Wirksamkeit der Therapie gegen ADHS, wobei die Therapie gegen ADHS eine kognitive Verhaltenstherapie und eine pharmakologische Therapie umfasst, die auf der Verabreichung von Psychostimulanzien basiert, die aus Methylphenidat und Amphetaminderivaten ausgewählt sind.

13. Verwendung des CpG-Dinukleotids nach Anspruch 12 in einem Subjekt mit einem DAT-9-Repeat-Allel.

14. Verwendung des CpG-Dinukleotids nach Anspruch 12 oder 13, wobei das Subjekt einen DAT 9/9- oder DAT 9/10-Genotyp aufweist.

## Revendications

1. Procédé permettant de déterminer la sévérité du trouble du déficit de l'attention avec hyperactivité (TDAH), ou permettant de déterminer l'efficacité d'une thérapie contre le TDAH, ledit procédé comprenant les étapes suivantes :
(i) purifier l'ADN génomique à partir d'un échantillon isolé ayant été obtenu d'un individu atteint du TDAH ou d'un individu suspecté d'être atteint du TDAH ;
(ii) faire réagir ledit ADN génomique avec du bisulfite ;
(iii) amplifier, de préférence par ACP, plus préférablement par RT-qPCR, au moins une partie de la région génomique comprise entre les coordonnées chromosomiques 1,392,790 et 1,445,430 du chromosome 5 humain comprenant au moins un dinucléotide CpG ou un îlot CpG, lesdites coordonnées étant calculées par rapport au génome humain 19 ;
(iv) mesurer le niveau de méthylation d'au moins un dinucléotide CpG ou d'au moins un îlot CpG compris dans la région amplifiée,
dans lequel ledit échantillon positif au TDAH est considéré comme sévère si le niveau de méthylation mesuré pour ledit CpG ayant la coordonnée 1,444,716 du chromosome 5 humain dans ledit échantillon est supérieur au niveau de méthylation mesuré pour un échantillon de référence du TDAH défini comme non sévère ;
dans lequel ladite thérapie est considérée comme efficace si le niveau de méthylation mesuré, dans un échantillon isolé d'un individu atteint du TDAH après le traitement thérapeutique, pour ledit au moins un dinucléotide CpG ou ledit au moins un îlot CpG est supérieur au niveau de méthylation mesuré dans le même échantillon avant ledit traitement, dans lequel ledit CpG ou îlot CpG est choisi parmi au moins un CpG ayant une coordonnée chromosomique choisie parmi : 1,444,713 et 1,444,692 du chromosome 5 humain, dans lequel ladite thérapie contre le TDAH comprend une thérapie cognitivo-comportementale et une thérapie pharmacologique basée sur l'administration de médicaments psychostimulants choisis parmi les dérivés du méthylphénidate et de l'amphétamine.

2. Procédé selon la revendication 1, dans lequel la mesure du niveau de méthylation selon l'étape (iv) est déterminée au moyen de la formule mC/(mC+C), dans laquelle mC désigne la cytosine méthylée et C la cytosine non méthylée.

3. Procédé selon la revendication 1 ou 2, dans lequel ledit échantillon biologique est un fluide biologique choisi dans le groupe constitué par : le sang, le sérum, l'urine, la salive et les selles.

4. Procédé selon la revendication 3, dans lequel ledit échantillon biologique est un écouvillon buccal.

5. Procédé selon l'une quelconque des revendications 1-4, dans lequel la quantité d'ADN génomique est comprise entre 100 et 1 000 nanogrammes ; plus préférablement est d'environ 500 nanogrammes.

6. Procédé selon l'une quelconque des revendications 1-5, dans lequel la région amplifiée est choisie dans le groupe constitué de la SEQ ID N° : 1-3.

7. Procédé selon l'une quelconque des revendications 1-6, en combinaison avec les scores d'au moins un questionnaire clinique et/ou le titre d'autoanticorps anti-DAT.

8. Procédé selon la revendication 7, dans lequel l'au moins un questionnaire clinique est un CGAS et/ou un Conners.

9. Procédé selon la revendication 7 ou 8, dans lequel le titre de l'autoanticorps anti-DAT est le titre sérique.

10. Utilisation du dinucléotide CpG de coordonnée chromosomique 1,444,716 du chromosome 5 humain comme biomarqueur de la sévérité clinique du TDAH.

11. Utilisation du dinucléotide CpG selon la revendication 10, chez un individu présentant un génotype homozygote DAT 10/10.

12. Utilisation du dinucléotide CpG ayant une coordonnée chromosomique choisie entre 1,444,713 et 1,444,692 du chromosome 5 humain comme biomarqueur de l'efficacité de la thérapie contre le TDAH, dans laquelle ladite thérapie contre le TDAH comprend une thérapie cognitivo-comportementale et une thérapie pharmacologique basée sur l'administration de médicaments psychostimulants choisis parmi les dérivés du méthylphénidate et de l'amphétamine.

13. Utilisation du dinucléotide CpG selon la revendication 12, chez un sujet présentant un allèle à 9 répétitions DAT.

14. Utilisation du dinucléotide CpG selon la revendication 12 ou 13, dans laquelle le sujet a un génotype DAT 9/9 ou DAT 9/10.
